Europäisches Patentamt

European Patent Office

Office européen des brevets

(19)

(11) Publication number: **0 062 136**
**B1**

(12) **EUROPEAN PATENT SPECIFICATION**

(45) Date of publication of patent specification: **25.09.85**

(21) Application number: **82100421.5**

(22) Date of filing: **21.01.82**

(51) Int. Cl.⁴: **C 07 C 139/14, C 07 C 143/02**

(54) **Process for the preparation of anhydrous alkane sulfonic acids.**

(30) Priority: **30.03.81 US 249139**

(43) Date of publication of application:
**13.10.82 Bulletin 82/41**

(45) Publication of the grant of the patent:
**25.09.85 Bulletin 85/39**

(84) Designated Contracting States:
**BE DE FR GB IT NL**

(56) References cited:
**DD-A- 146 045**
**US-A-3 729 507**
**US-A-4 035 242**

(73) Proprietor: **PENNWALT CORPORATION**
**Pennwalt Building Three Parkway**
**Philadelphia Pennsylvania 19102 (US)**

(72) Inventor: **Guertin, Roland Maurice**
**31019 West Jefferson**
**Rockwood Michigan 48173 (US)**

(74) Representative: **Kraus, Walter, Dr. et al**
**Patentanwälte Kraus Weisert & Partner Thomas-Wimmer-Ring 15**
**D-8000 München 22 (DE)**

The file contains technical information submitted after the application was filed and not included in this specification

Courier Press, Leamington Spa, England.

EP 0 062 136 B1

## Description

### Background of the invention

The present invention relates to the production of anhydrous organic sulfonic acids, and more particularly, to methods for removing water from aqueous solutions of alkane sulfonic acids.

Organic sulfonic acids may be produced by a variety of processes. For example, aliphatic sulfonic acids may be prepared by methods including the following: (1) the oxidation of thiols, (2) the reaction of sodium sulfite with certain alkyl halides, and (3) the oxygen-induced, anti-Markownikoff addition of sodium bisulfite to alkenes. (See J. Roberts and M. Caserio, *Modern Organic Chemistry* (W. A. Benjamin, Inc., New York, 1967) at pages 520, 523—24). Alkyl sulfonic acids may be prepared by the oxidation of alkyl mercaptans or dialkyl disulfides with hydrogen peroxide in the presence of alkyl sulfonic acids as disclosed in U.S. Patent 4,239,696 to Schreyer et al. In addition, more complex sulfonic acids have been synthesized such as by the processes disclosed in U.S. Patent 3,130,221 to Oesterling, and U.S. Patent 2,204,210 to Farlow. The purity of alkane sulfonic acids produced by the above described methods ranges from about 70% to about 90%.

For some applications it is desirable to have alkane sulfonic acids that are substantially anhydrous, for example, acids that contain less than 3% water. Previous methods for obtaining alkane sulfonic acids with a reduced water content have involved distillation of an aqueous mixture of the acid at reduced pressures, e.g. 5—100 mm of mercury, with subsequent collection of the anhydrous material as an overheads product. U.S. Patent 4,035,242 to Brandt describes a distillative purification process for alkane sulfonic acids.

In a distillative purification process the aqueous alkane sulfonic acid solution is normally heated to a temperature sufficient to distill off the acid and to collect it as an overheads product. Such heating of the solution may lead to the formation of side products (including degradation products), e.g., esters. These esters may be formed, for example, by acid reacting with alcohol impurities. Such side products are undesirable as they reduce the yield of alkane sulfonic acid. Additionally the time, energy, and supporting equipment needed to maintain reduced pressures during distillation may be considerable and expensive. Also, anhydrous alkane sulfonic acids purified by the above described method may also have an objectionable odor.

### Summary of the invention

The present invention provides a novel method for treating aqueous solutions containing an alkane sulfonic acid to produce a substantially anhydrous alkane sulfonic acid product. The method of this invention may be used to produce alkane sulfonic acids having less than 3% water and, more particularly, having as little as 0.5% to 1.0% water. The method of this invention may be practiced to produce a substantially anhydrous alkane sulfonic acid product without the formation of objectionable odors or side products.

In accordance with the preferred embodiment of the present invention, a regulated stream of moisture absorbing gas, such as air, is brought into contact with an aqueous solution of alkane sulfonic acid to remove water from that solution. In practicing the method of this invention it is also preferred that the solution be heated to temperatures sufficient to release water(s) of hydration, but below the boiling point of the alkane sulfonic acid to be collected. As the process continues, a substantially anhydrous alkane sulfonic acid product is formed which may be collected as a bottoms product.

The method of this invention may be practiced without requiring the pressure modifications normally attendant to vacuum distillation methods.

The method of this invention is particularly useful for alkane sulfonic acids containing from one to about twelve carbon atoms, preferably from one to about eight carbon atoms. The alkyl group may be straight chain or branched, unsubstituted or substituted with a member from the group comprising —OH, —Cl, —Br, —F, —NH$_2$, —SO$_3$H, —SO$_2$Cl, or —SO$_3$R', where R' is C$_1$—C$_8$.

Thus, a primary object of this invention is the provision of an improved method for removing water from alkane sulfonic acids without the formation of objectionable side products.

Another object of this invention is the provision of a method for removing water from alkane sulfonic acids which may be utilized at atmospheric pressure.

A further object of this invention is the provision of alkane sulfonic acids having a water content as low as from about 0.5% to about 1.0%.

Further objects of the present invention will become apparent from the following more detailed description of the method of this invention.

### Brief description of the drawings

Figure 1 is a diagrammatic view of a laboratory scale apparatus for use in treating alkane sulfonic acids in accordance with the method of the present invention; and

Figure 2 is a diagrammatic view of an alternate laboratory scale apparatus which is suitable for use in practicing the method of this invention.

### Detailed description

It has been found that an alkane sulfonic acid bottoms product having less than about 3% water, and, more particularly, having from about 1.0% to about 0.5% water, may be obtained by performing a method which includes contacting a stream of moisture absorbing gas (e.g. air) with an aqueous alkane sulfonic acid solution. Thus, as used herein, the term "anhydrous" or "substantially anhydrous" alkane sulfonic acid refers to an acid of that type which does not contain more than about 3% water. Preferable the aqueous alkane sulfonic acid and/or the stream of moisture absorbing gas is warmed to a temperature sufficient to cause water(s) of hydration to be freed from the alkane sulfonic acid, but below the boiling point of the acid. The method of this invention be useful in removing water from such aqueous solutions, it has been found that air works well with the method of this invention. Other moisture absorbing gases presently believed suitable for use in practicing the method of the present invention include nitrogen, hydrogen, carbon dioxide, and mixtures thereof.

Due to the corrosive nature of some alkane sulfonic acids, it is preferred that the types of apparatus used in practicing the method of this invention be constructed of materials that resist the corrosive effects of these substances. Glass, tantalum, and inert plastic are examples of such materials.

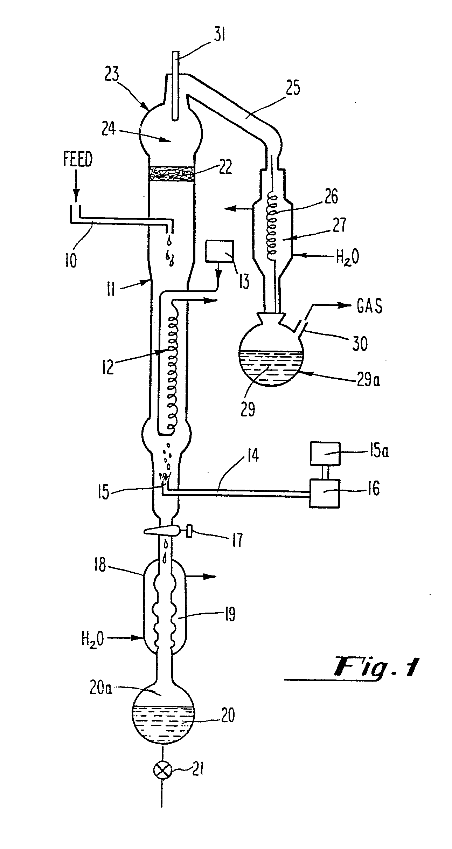

The process of this invention may be more clearly understood by referring to Figure 1, which depicts a specific example of an apparatus which may be used to practice the method of this invention. Feed stock of an alkane sulfonic acid is introduced at a preselected feed rate by means of a delivery conduit 10 at the top of a column 11. Once introduced into column 11, the feed stock is warmed by contact with an enclosed coil of oil 12, which is positioned within column 11. The oil within coil 12 may be heated and circulated by an external pumping means 13, such as a centrifugal pump. As the feed stock containing alkane sulfonic acid passes downwardly through column 11, it is also contacted by a stream of moisture absorbing gas 15, e.g. air, which has been introduced into the column 11, at a preselected rate, through conduit 14 from a reservoir 15a. The moisture absorbing gas 15 may be introduced at ambient temperature, but more preferably the gas has been preheated to a preselected temperature by an external heating means 16. During its contact with the moisture absorbing gas, the alkane sulfonic acid releases water to the gas to form a reduced water content alkane sulfonic acid product. As the reduced water content alkane sulfonic acid product passes further down column 11 it passes through a stop cock 17 to a cooling condenser 18 which is cooled by a water jacket 19. After cooling the alkane sulfonic acid product 20 passes into a collection vessel 20a. In a continuous system, the reduced water content alkane sulfonic acid product may be removed periodically through a valve 21.

While contacting the alkane sulfonic acid, the moisture absorbing gas 15 moves upwardly in a counter-current direction with respect to the flow of the feed stock of alkane sulfonic acid. While passing through column 11, the gas 15 becomes a gaseous mixture which further comprises water and trace amounts of alkane sulfonic acid. This gaseous mixture may also contain other materials which have been removed from the feed stock such as $H_2SO_4$ and esters of the acid. The gaseous mixture travels upwardly into a demister 22, where much of the liquid material is removed. The gaseous mixture then continues to travel upwardly through a disengaging area within a glass bulb 23 which defines space 24 where some condensation may take place. A thermometer 31 is shown as a means for monitoring the temperature of its associated portion of the system. The gaseous mixture then travels through a side arm 25 to condenser 26 which is cooled by a water jacket 27. Liquid 29 which is condensed from the gaseous mixture is collected in container 29a and may be used as feed stock in the process of the present invention to recover any remaining alkane sulfonic acid contained therein. The residual gas is either recycled into the system or vented to the outside by means of a vent 30.

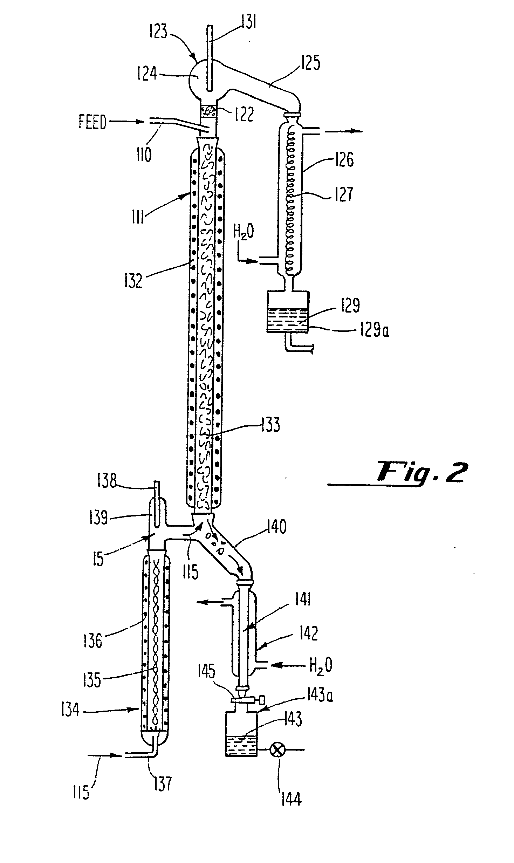

The apparatus described in Figure 1 may be used with column 11 empty or filled. In a preferred method for using the apparatus diagramed in Figure 2 for treating MSA, stop cock 17 is initially closed and column 11 is filled to a preselected level (preferably a major portion of the column) with anhydrous MSA. This column containing anhydrous MSA is then warmed to a preselected temperature, preferably about 145°C to about 155°C, and more preferably about 150°C. After this heating has been accomplished, the aqueous solution of MSA is introduced into the column at a preselected rate as previously described in Figure 1. Air (used as the moisture absorbing gas) is introduced through conduit 14 as previously described in Figure 1, and is bubbled through the column 11. Stop cock 17 is at least partially opened to allow collection of a reduced water content MSA product at a preselected rate. The rest of the process proceeds as previously described in Figure 1.

Figure 2 illustrates a different apparatus suitable for use in performing the method of this invention. A stream of moisture absorbing gas 115 is introduced at a preselected rate through a tube 137 into a heating column 136. The heating column 136 comprises an electrical preheating means 134 and internally disposed nichrome helices 135. After passing through the heating column 136, the moisture absorbing gas 115 then enters head 139, the internal temperature of which is monitored by thermometer 138. Head 139 keeps the heating column 136 out of direct alignment with a process column 111 and prevents any process column liquid material from entering heating column 136. The stream of moisture absorbing gas 115 then enters the bottom of process column 111, which contains ceramic Beryl saddles 133. The column 111 is heated by an external electrical coil 132 (shown in a sectional view here). As the stream of moisture absorbing gas 115 moves upwardly through the column 111, it countercurrently engages feed stock of an alkane sulfonic acid which is introduced by means of delivery conduit 110 at the top of column 111. After contacting the moisture absorbing gas 115 within column 111, the resultant reduced water content alkane sulfonic acid product flows downwardly through side arm 140 positioned at the base of column 111, and then into a condenser 141 which is cooled by a water jacket 142. The alkane sulfonic acid product 143 flows through stop cock 145 which is adjusted to be open enough to allow collection of the alkane sulfonic acid 143, but not so open as to allow substantial escape of the moisture absorbing gas through this route. This alkane sulfonic acid product 143 is then collected in a container 143a. In a continuous system, valve 144 may be used to periodically remove the alkane sulfonic acid product 143.

While passing through process column 111, the moisture absorbing gas 115 becomes a gaseous mixture which comprises water and trace amounts of alkane sulfonic acid. This gaseous mixture may also contain other materials as explained above. The gaseous mixture travels upwardly into a demister 122 where much of the liquid material is removed. The gaseous mixture then continues to travel upwardly through a disengaging area 123 which defines a space 124 where some condensation may occur. The gaseous mixture then travels through a sidearm 125 to condenser 127 which is cooled by a water jacket 127. Preferred examples of types of condensers suitable for use at this step in the method of this invention include those having a large condensing area, such as a Graham condenser. The liquid 129 which is condensed from the gaseous mixture is collected in container 129a, and may be used as feed stock in the process of the present invention to recover any remaining alkane sulfonic acid contained therein.

In practicing the method of this invention, selection and control of parameters such as temperatures and flow rates are important factors in optimizing residence time of the alkane sulfonic acid in the process column, and in maximizing the efficiency of a particular system. For example, increasing the temperature of the column may decrease the residence time needed for removing water from an aqueous solution of an alkane sulfonic acid, but may increase the production of degradation products; however, increasing the feed rate for the aqueous solution of the acid and/or the gas may reduce the residence time of the acid in the process column, thereby minimizing the formation of degradation products and allowing for faster production of a reduced water content alkane sulfonic acid. By optimizing temperatures and flow rates for the feed stock and the moisture absorbing gas, one may obtain an anhydrous alkane sulfonic acid as a product on a first run of the process. If the temperature and flow rates are not carefully selected and controlled, the reduced water content alkane sulfonic acid product collected may have to be recycled through the process to obtain an anhydrous product. These temperatures and flow rates will vary depending on the type of alkane sulfonic acid to be processed. It will be appreciated by those skilled in the art that temperatures and flow rates will have to be adjusted and optimized as column diameters and column packings are varied.

The following examples are offered as illustrative of this invention but should not be construed as limitations thereon. Unless otherwise specified, all percent compositions are on weight basis.

Examples I—IV—Methane sulfonic acid

The following examples were run using the apparatus shown in Figure 2 and the process explained therein. The data for these examples are listed in Table I. Air preheater temperature was measured by means of thermometer 138, and stripper overhead temperature was measured by thermometer 131. The moisture absorbing gas used here was air, and the flow rate in liters/min. (l./min.) was measured by a wet testmeter. The feed stock used comprised methanesulfonic acid (MSA) of varying degrees of purity. The diameter of the processing column was 1¼ inches. The flow rate of the feed stock was measured in milliliters/min. (ml./min.) by using a proportioning pump which had been calibrated with water. Analyses for MSA and water were done for the bottoms product collected and the aqueous overheads material collected. This data is also found in Table I.

Example I

A 90% methanesulfonic acid (MSA) solution was prepared by diluting a quantity of 100% MSA with enough water to give a 90% concentration of MSA. Temperature ranges for the air preheater and stripper overhead are given in Table I. The flow rate for air and feed rate of MSA feed stock are also given in Table I. A product with substantially anhydrous MSA and having a purity of 99.57% was obtained. The aqueous overheads material was collected and analyzed for water and MSA content with % MSA listed in Table I.

Example II

The feed stock used here was 70% MSA reactor crude, i.e. the MSA was in a form as it comes from the synthesis of MSA without any further purification step. Temperatures and flow rates are listed in Table I. An anhydrous product of 99.85% purity was obtained.

Example III

The feed stock was 70% MSA which had been pretreated by a stripping process step (PF 1759, Pennwalt Corp.). Temperatures and flow rates are listed in Table I. An anhydrous product of 99.66% purity was obtained.

Example IV

MSA of 96.45% purity was obtained from a previous run. Reduction of the water content was desired so this material was recycled through the system of Figure 2. Temperatures and flow rates are listed in Table I. An anhydrous product of 99.88% purity was obtained.

As explained above, temperatures and flow rates must be controlled and optimized with respect to the particular alkane sulfonic acid to be treated and the types of columns (including diameter and packing) used. For example, in the system described in Figure 2 as used for treating MSA, the temperature of the MSA feed stock was in the range of about 82°C to about 135°C, and the flow rate for the MSA feed stock was from about 5 ml./min. to about 14 ml./min. Air was used as the moisture absorbing gas. It was fed into the system at a rate of about 9 l./min., and preheated to a temperature from about 86°C to about 146°C. The regulation of temperature and flow rates is important for allowing adequate wetting of surface area in the column and optimizing the residency time of the alkane sulfonic acid in the column so that water may be removed from the acid. For example, in the system described in Figure 2 as used in treating MSA, it is preferred that the residency time be at least about 10 to about 30 seconds, and more preferably about 20 seconds.

It will be appreciated by those skilled in the art that other factors may affect optimization of practicing the process of this invention, including:

the height of the process column, the type and amount of packing used in the process column, and the tightness with which the column is packed.

While the method of this invention has been described as useful for removing water from alkane sulfonic acids it is to be understood that the process of this invention may also be useful for reducing the water content of other organic sulfonic acids such as sulfonic acids containing various other aliphatic or alicyclic moieties.

While the previous types of apparatus and examples have been explained in some detail, the method of this invention is not limited to such an apparatus and may be practiced with modifications and/or other equipment. For example, as an alternate embodiment a column stripper such as the type currently used with live steam may be employed. By using a moisture absorbing gas and no live steam, and controlling the temperature parameter and feed rates for the moisture absorbing gas and alkane sulfonic acid feed stock, an anhydrous product may be obtained without pretreating the crude alkane sulfonic acid mixture. Other apparatus may also be used which is within the spirit and scope of this invention.

TABLE I

| Example number | Feed stock | Air preheater Temp. | Stripper overhead Temp. | Air flow liters/min. | MSA feedstock ml./min. | Bottoms product % MSA | Aqueous overheads product | |
|---|---|---|---|---|---|---|---|---|
| | | | | | | | Approximate volume | % MSA |
| I | 90% MSA | 86—86°C | 122—135°C | 9 | 10 | 99.57 | 30 ml | 22.35 |
| II | 70% MSA reactor crude | 85—86°C | 124—127°C | 9 | 5 | 99.85 | 45 ml | not done |
| III | 70% MSA | 140—145°C | 82—120°C | 9 | 7 | 99.66 | 50 ml | 4.33 |
| IV | 96.45% MSA (recycled) | 136—146°C | 115—135°C | 9 | 14 | 99.88 | 50 ml | 26.21 |

## Claims

1. A method for treating an aqueous alkane sulfonic acid solution to remove water therefrom, said method comprising:

maintaining an aqueous alkane sulfonic acid solution at a preselected temperature below the boiling point of said alkane sulfonic acid solution but sufficient to cause a separation between an alkane sulfonic acid and water(s) of hydration of said alkane sulfonic acid, when contacted by a moisture absorbing gas;

contacting said aqueous alkane sulfonic acid solution with moisture absorbing gas until substantially anhydrous alkane sulfonic acid is produced as a bottoms product; and

recovering said substantially anhydrous alkane sulfonic acid bottoms product.

2. The method of Claim 1 wherein said moisture absorbing gas is air.

3. The method of Claim 1 wherein said aqueous alkane sulfonic acid comprises at least 70% of said alkane sulfonic acid solution.

4. The method of Claim 1 wherein said method is substantially continuous.

5. The method of Claim 1 wherein said aqueous alkane sulfonic acid solution comprises an alkane sulfonic acid having from one to twelve carbon atoms.

6. The method of Claim 5 wherein said alkane sulfonic acid is methanesulfonic acid and said preselected temperature of said alkane sulfonic acid solution is from about 80°C to about 160°C.

7. The method of Claim 1 wherein said contacting of said aqueous alkane sulfonic acid solution with said moisture absorbing gas comprises the steps of introducing said aqueous alkane sulfonic acid solution into a column at a preselected temperature and feed rate and introducing said moisture absorbing gas into said column at a preselected rate in a predetermined direction relative to a path of said aqueous alkane sulfonic acid solution.

8. The method of Claim 7 wherein said predetermined direction is substantially counter-current to said path of said aqueous alkane sulfonic acid solution.

9. The method of Claim 7 wherein said aqueous alkane sulfonic acid solution comprises an alkane sulfonic acid having from one to twelve carbon atoms.

10. The method of Claim 9 wherein said alkane sulfonic acid has from one to eight carbon atoms.

11. The method of Claim 9 wherein said alkane sulfonic acid is methanesulfonic acid.

12. The method of Claim 1 wherein said contacting of said aqueous alkane sulfonic acid solution with said moisture absorbing gas comprises introducing said aqueous alkane sulfonic acid solution into a packed column stripper apparatus at a preselected temperature and feed rate and introducing said moisture absorbing gas into said column stripper at a preselected flow rate substantially counter-current to a path of said alkane sulfonic acid solution until substantially anhydrous alkane sulfonic acid is produced.

## Patentansprüche

1. Verfahren zur Behandlung einer wäßrigen Alkansulfonsäurelösung zur Entfernung von Wasser daraus, dadurch gekennzeichnet, daß man

eine wäßrige Alkansulfonsäurelösung bei einer vorgewählten Temperatur unterhalb des Siedepunkts der genannten Alkansulfonsäurelösung, die aber ausreichend ist, um eine Trennung zwischen einer Alkansulfonsäure und Hydratationswasser der genannten Alkansulfonsäure bei der Kontaktierung mit einem feuchtigkeitsabsorbierenden Gas zu bewirken, hält,

die genannte wäßrige Alkansulfonsäure mit dem feuchtigkeitsabsorbierenden Gas kontaktiert, bis im wesentlichen wasserfreie Alkansulfonsäure als Bodenprodukt erzeugt wird, und daß man

das genannte, im wesentlichen wasserfreie Alkansulfonsäurebodenprodukt gewinnt.

2. Verfahren nach Anspruch 1, dadurch gekennzeichnet, daß das genannte feuchtigkeitsabsorbierende Gas Luft ist.

3. Verfahren nach Anspruch 1, dadurch gekennzeichnet, daß die genannte wäßrige Alkansulfonsäure mindestens 70% der genannten Alkansulfonsäurelösung umfaßt.

4. Verfahren nach Anspruch 1, dadurch gekennzeichnet, daß das genannte Verfahren im wesentlichen kontinuierlich ist.

5. Verfahren nach Anspruch 1, dadurch gekennzeichnet, daß die genannte wäßrige Alkansulfonsäurelösung eine Alkansulfonsäure mit 1 bis 12 Kohlenstoffatomen enthält.

6. Verfahren nach Anspruch 5, dadurch gekennzeichnet, daß die genannte Alkansulfonsäure Methansulfonsäure ist und daß die genannte vorgewählte Temperatur der genannten Alkansulfonsäurelösung etwa 80°C bis etwa 160°C ist.

7. Verfahren nach Anspruch 1, dadurch gekennzeichnet, daß die Kontaktierung der genannten wäßrigen Alkansulfonsäurelösung mit dem genannten feuchtigkeitsabsorbierenden Gas die Stufen der Einführung der genannten wäßrigen Alkansulfonsäurelösung in eine Säule bei vorgewählter Temperatur und Beschickungsgeschwindigkeit und die Einführung des genannten feuchtigkeitsabsorbierenden Gases in die genannte Säule mit vorgewählter Geschwindigkeit in vorgewählter Richtung relativ zu dem Weg der genannten wäßrigen Alkansulfonsäurelösung umfaßt.

8. Verfahren nach Anspruch 7, dadurch gekennzeichnet, daß die genannte vorgewählte Richtung im wesentlichen gegenläufig zu dem genannten Weg der genannten wäßrigen Alkansulfonsäurelösung ist.

9. Verfahren nach Anspruch 7, dadurch gekennzeichnet, daß die genannte wäßrige Alkansulfonsäurelösung eine Alkansulfonsäure mit 1 bis 12 Kohlenstoffatomen enthält.

10. Verfahren nach Anspruch 9, dadurch gekennzeichnet, daß die genannte Alkansulfonsäure 1 bis 8 Kohlenstoffatome aufweist.

11. Verfahren nach Anspruch 9, dadurch gekennzeichnet, daß die genannte Alkansulfonsäure Methansulfonsäure ist.

12. Verfahren nach Anspruch 1, dadurch gekennzeichnet, daß die genannte Kontaktierung der genannten wäßrigen Alkansulfonsäurelösung mit dem genannten feuchtigkeitsabsorbierenden Gas die Einführung der genannten wäßrigen Alkansulfonsäurelösung in eine gepackte Säulenabstreifvorrichtung bei vorgewählter Temperatur und Beschikkungsgeschwindigkeit und die Einführung des genannten feuchtigkeitsabsorbierenden Gases in die genannte Säulenabstreifeinrichtung bei vorgewählter Fleißgeschwindigkeit im wesentlichen im Gegenstrom zu einem Weg der genannten Alkansulfonsäurelösung umfaßt, bis im wesentlichen wasserfreie Alkansulfonsäure erzeugt wird.

**Revendications**

1. Procédé de traitement d'une solution aqueuse d'acide alcanesulfonique pour en éliminer l'eau, ledit procédé consistant:

à maintenir une solution aqueuse d'acide alcanesulfonique à une température prédéterminée au-dessous du point d'ébullition de ladite solution d'acide alcanesulfonique, mais suffisante pour provoquer une séparation entre un acide alcanesulfonique et l'eau d'hydratation dudit acide alcanesulfonique, au contact d'un gaz absorbant l'humidité;

à faire entrer ladite solution aqueuse d'acide alcanesulfonique en contact avec un gaz absorbant l'humidité jusqu'à ce que de l'acide alcanesulfonique pratiquement anhydre ait été formé comme produit de queues; et

à recueillir le produit de queues formé d'acide alcanesulfonique pratiquement anhydre.

2. Procédé suivant la revendication 1, dans lequel le gaz absorbant l'humidité est l'air.

3. Procédé suivant la revendication 1, dans lequel l'acide alcanesulfonique aqueux constitue au moins 70% de ladite solution d'acide alcanesulfonique.

4. Procédé suivant la revendication 1, ledit procédé étant pratiquement continu.

5. Procédé suivant la revendication 1, dans lequel ladite solution aqueuse d'acide alcanesulfonique comprend un acide alcanesulfonique ayant 1 à 12 atomes de carbone.

6. Procédé suivant la revendication 5, dans lequel ledit acide alcanesulfonique est l'acide méthanesulfonique et ladite température prédéterminée de ladite solution d'acide alcanesulfonique va d'environ 80 à environ 160°C.

7. Procédé suivant la revendication 1, dans lequel la mise en contact de ladite solution aqueuse d'acide alcanesulfonique avec ledit gaz absorbant l'humidité comprend les étapes d'introduction de ladite solution aqueuse d'acide alcanesulfonique dans une colonne à une température et à une vitesse d'alimentation prédéterminées et l'introduction du gaz absorbant l'humidité dans ladite colonne à une vitesse prédéterminée dans une direction prédéterminée par rapport à un trajet de ladite solution aqueuse d'acide alcanesulfonique.

8. Procédé suivant la revendication 7, dans lequel ladite direction prédéterminée est pratiquement à contre-courant avec ledit trajet de la solution aqueuse d'acide alcanesulfonique.

9. Procédé suivant la revendication 7, dans lequel la solution aqueuse d'acide alcanesulfonique comprend un acide alcanesulfonique ayant 1 à 12 atomes de carbone.

10. Procédé suivant la revendication 9, dans lequel l'acide alcanesulfonique comprend 1 à 8 atomes de carbone.

11. Procédé suivant la revendication 9, dans lequel l'acide alcanesulfonique est l'acide méthanesulfonique.

12. Procédé suivant la revendication 1, dans lequel la mise en contact de la solution aqueuse d'acide alcanesulfonique avec le gaz absorbant l'humidité consiste à introduire ladite solution aqueuse d'acide alcanesulfonique dans un appareil rectificateur à colonne garnie à une température et une vitesse d'alimentation prédéterminées et à introduire ledit gaz absorbant l'humidité dans ledit rectificateur à colonne à une vitesse d'écoulement prédéterminée pratiquement à contre-courant avec un trajet de ladite solution d'acide alcanesulfonique jusqu'à ce qu'un acide alcanesulfonique pratiquement anhydre ait été produit.

*Fig.1*

_Fig. 2_